# EUROPEAN PATENT APPLICATION

(11) **EP 1 418 428 A1**
(43) Date of publication of application: **12.05.2004**
(21) Application number: 02292787.5
(22) Date of filing: 07.11.2002
(51) Int. Cl.: G01N 33/50, G01N 33/68

(54) **Method to provide natural therapeutic agents with high therapeutic index**

(71) Applicant: GenOdyssee, 91974 Courtaboeuf (FR)
(72) Inventor: Escary, Jean-Louis, 78150 Le Chesnay (FR)
(74) Representative: Santarelli

(57) **Abstract**

A method for providing new therapeutic agent(s) that comprises the following steps:
a) selecting at least one polypeptide encoded by a natural allelic variant of one preselected gene with therapeutic potential and/or of at least one related gene(s) thereof;
b) determining the therapeutic index of the polypeptide(s) selected in step a); and
c) retaining as a therapeutic agent(s), the polypeptide(s) selected in step a), whose therapeutic index, as determined in step b), is higher than a therapeutic index of reference.

## Description

### BACKGROUND OF THE INVENTION

### Field of the invention

The present invention relates to a method for providing therapeutic agents in particular polypeptides having a high therapeutic index, as well as polynucleotides encoding said polypeptides.

### Background art

The aim of drug discovery is to develop safe and effective drug. Many methods are currently used in this purpose. Generally, after having identified and validated a target, a large number of molecules are screened against these targets to identify those molecules that have the potential to progress through the lengthy and expensive drug development process.

To develop new therapeutic agents from peptide and/or protein compounds, different approaches may be followed. One possible approach is to strategically design a library of peptide compounds by introducing random mutation on positions of the molecule that are liable to improve functionality. Alternatively, random mutations may be introduced throughout the gene using for example error-prone PCR or an *Escherichia coli* strain lacking DNA repair mechanisms. Another interesting approach consists of generating sequence diversity by DNA shuffling (Stemmer (1994), Nature 370, 389-391 and Coco *et al.* (2001), Nature Biotechnology 19, 354-359). This method consists in randomly amplifying and fragmenting related DNA sequences, then reassembling the fragments using DNA polymerase in a self-priming fashion. The resulting chimeric molecules are selected and can be bred again, accumulating multiple beneficial mutations. This method belongs to *in vitro* or *in vivo* recombination based techniques such as directed evolution technologies, which are techniques well known to the one skilled in the art. In complement of these methods, computational approaches may also be suitable to increase rational protein design and reduce the number of molecules on which to perform the experimental tests (Hellinga (1998), Nat. Struct. Biol. 5:525-527; DeGrado *et al.* (1999), Annu. Rev. Biochem. 68:779-819; Gordon *et al.* (1999) Curr. Opin. Struct. Biol. 9: 509-513; Janin (1996) Proteins 25:438-445).

Each of the methods described above has its own advantages and limitations. As limitations, we can quote the necessity to evaluate a large number of molecules, the necessity to dispose of sufficient structural information on the gene or the product of said gene, the necessity to generate a high and often useless number of related gene sequences and the necessity for a rational lead optimisation. Furthermore, these methods generate non-naturally occurring proteins, which are susceptible to induce unexpected side effects in the patients, for instance immunogenicity.

In the past years, natural genetic polymorphisms (which include natural allelic variants of a gene) and polypeptides encoding thereby were known to be useful mainly for:
- linkage analysis, evolutionary studies, forensics.
- establishing phylogenetic relationships between different species.
- generally determining approximate levels of DNA sequence diversity both within and between living individuals.
- analysing genome diversity.
- identifying disease-causing gene mutations, genetic marker development, and the like (diagnostic/prognosis of a disease or pathology).
- providing large numbers of low mutating genetic markers for use in gene mapping.
- using forensics applications such as DNA fingerprinting.
- targets for drug discovery and drug development.

Until now, natural allelic variants of genes which are frequently found in a given population, have not been regarded by one skilled in the art as a source for providing a significant number of therapeutic agents, in particular therapeutic agents having high therapeutic efficiency.

Indeed, it could not be expected that polypeptides encoded by natural allelic variants of a same gene, differing by as little as only one amino acid, could have significant different pharmacological properties and/or pharmacological profiles.

This was even less expected for proteins like cytokines, which show pleiotropic activity.

It is emphasized here on the fact that, if it might happen that one specific natural allelic variant was tested in a biological assay, it has not yet been proposed to use as many as possible of such variants to determine among them those which can actually be useful as therapeutic agents.

It was still less proposed a method permitting to simply select and provide, among polypeptides encoded by several natural allelic variants, those useful as therapeutic agents.

Therefore, one of the main purposes of the present invention is a new method for providing new therapeutic agent(s), which method overcomes some or all drawbacks of the above-mentioned known methods therefor.

### BRIEF SUMMARY OF THE INVENTION

A first object of the invention is a method for providing new therapeutic agent(s), characterized in that it comprises the following steps:
a) selecting at least one polypeptide encoded by at least one natural allelic variant(s) of one preselected gene with therapeutic potential and/or of at least one related gene(s) of said preselected gene with therapeutic potential;
b) determining the therapeutic index of the polypeptide(s) selected in step a); and
c) retaining as a therapeutic agent(s), the polypeptide(s) selected in step a), whose therapeutic index, as determined in step b), is higher than a therapeutic index of reference.

The natural allelic variants which encode the polypeptides selected in step a), can be a natural allelic variant of any preselected gene with therapeutic potential, as defined hereunder, or any gene that belongs to the same gene family as said preselected gene with therapeutic potential. In other words, the method of the present invention enables to identify polypeptides encoded by natural allelic variants of a known gene, genes of the same gene family as said known gene, or both a known gene and genes of the same gene family of said known gene.

The natural allelic variants of a gene usually comprise one variant that is relatively frequent in a given population and some others that are less frequent or even rare, to an extend that one skilled in the art would usually consider that they have no significant influence on the biology of the whole population. Nevertheless, these natural allelic variants can individually display different activity profile. This may be due to unexpected differences in stability or structure. Furthermore, the fact that these natural allelic variants have been fixed by certain individuals in the population suggests that they are likely to be safely tolerated by the organism.

The method of the invention may be applied to the natural allelic variants of one single gene that can be either a preselected gene with therapeutic potential or any gene belonging to the same gene family as said preselected gene. Preferably, at least 2 polypeptides, and more preferably at least 4 polypeptides encoded by natural allelic variants of a preselected gene or of one related gene, are selected in step a).

The maximum number of polypeptides that can be carried out in the method according to the present invention mainly depends on the number of identified natural allelic variants encoding for such peptides

When the preselected gene with therapeutic potential belongs to a gene family, the method of the invention may be preferably applied to natural allelic variants of several genes belonging to the same gene family as said preselected gene as well as to natural allelic variants of said preselected gene.

The natural allelic variants may originate from different species, but preferably originates from the same species, more preferably from the human species.

In another aspect of the invention, for each polypeptide obtainable according to the method of the invention, the therapeutic index is established by first submitting said polypeptides to at least two activity tests, second attributing a value in direct relation with the results of said activity tests, and finally determining the therapeutic index from the obtained values.

According to the invention, in step c) are retained as therapeutic agent(s) only the polypeptide(s) selected in step a) whose therapeutic index is higher than the therapeutic index of reference.

In a preferred aspect of the invention, the polypeptides with the highest or second highest therapeutic index are those selected as therapeutic agent(s). Thus, the preferred therapeutic agents of the invention are the polypeptides having the highest or second highest therapeutic index, it being understood that their therapeutic index is also higher than that of the reference.

Also, the method of the invention may be used to provide new therapeutic agents with new pharmacological profiles, which may eventually become available for the development of new therapeutic applications.

Another object of the invention concerns the use of the polypeptides provided by the method described herein, as well as the polynucleotides encoding said polypeptides, as well as their derivatives, as therapeutic agents for the manufacture of a medicine to be administered to a patient in need thereof.

### BRIEF DESCRIPTION OF THE DRAWINGS

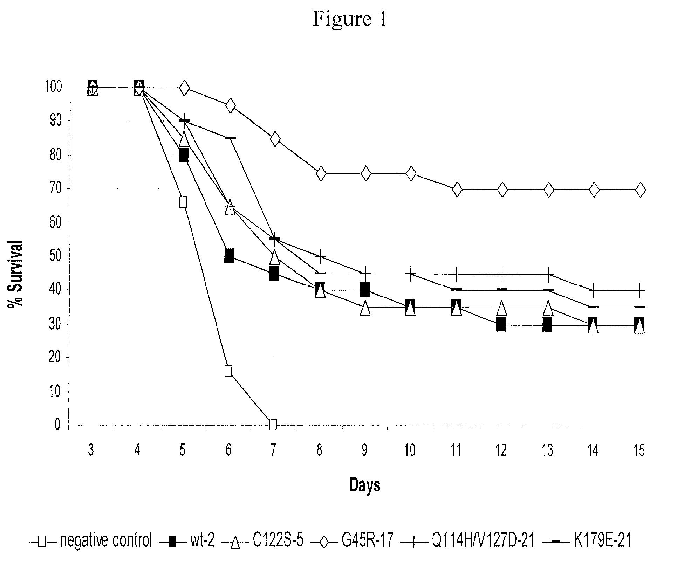

Figure 1 represents the survival rate of mice previously infected by EMCV virus and treated with one of five polypeptides encoded by natural allelic variants of genes belonging to the IFNα gene family, or those which received excipient only. In this figure, the abscissas correspond to the time of survival (days) and the ordinates correspond to the relative survival rate of EMCV infected mice. The interferons tested are: C122S IFNα-5 (Δ), G45R IFNα-17 (◇), Q114H and V127D IFNα-21 (+), and K179E IFNα-21 (-), wild type IFNα-2 (■). A negative control (□) corresponding to excipient only is also presented.

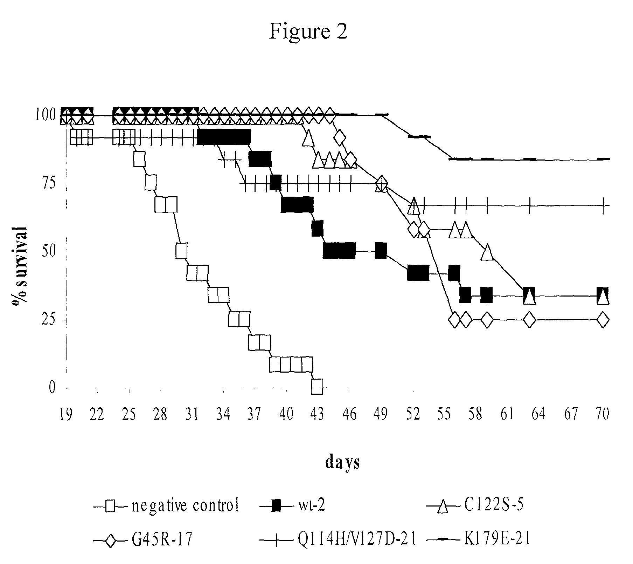

Figure 2 represents the survival rate of mice previously inoculated with malignant Friend erythroleukemia cells (FLC) and treated with one of five polypeptides encoded by natural allelic variants of genes belonging to the IFNα gene family, or those which received excipient only. In this figure, the abscissas correspond to the time of survival (days) and the ordinates correspond to the relative survival rate of FLC inoculated mice. The interferons tested are: C122S IFNα-5 (Δ), G45R IFNα-17 (◇), Q114H and V127D IFNa-21 (+), and K179E IFNα-21 (-), wild type IFNα-2 (■). A negative control (□) corresponding to excipient only is also presented.

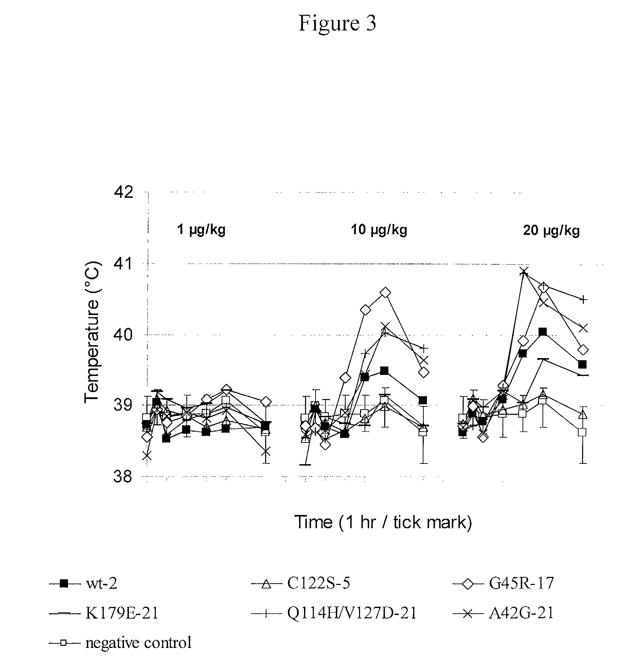

Figure 3 represents the effect of subcutaneous administration of one of five polypeptides encoded by natural allelic variants of genes belonging to the IFNα gene family on body temperature of Rhesus monkeys. Three doses of interferons have been tested: 1, 10 or 20 µg/kg. In this figure, the abscissas correspond to the time after interferon administration (1 tip mark represents one hour) and the ordinates correspond to body temperature (°C). The interferons tested are: C122S IFNα-5 (Δ), G45R IFNα-17 (◇), Q114H and V127D IFNα-21 (+), and K179E IFNα-21 (-), wild type IFNα-2 (■). A negative control (□) corresponding to excipient only is also presented.

### DETAILED DESCRIPTION OF THE INVENTION

The following terms, used in the present description have the meanings given here below.

A **"therapeutic agent"** designates a substance that can be used in a medical treatment.

A **"preselected gene with therapeutic potential"** designates a known gene encoding at least one protein, said polypeptide being selected in the group consisting of:
- polypeptides developed in the industry as a therapeutic agent and which are already used on the market as therapeutic agents in at least one therapeutic application;
- polypeptides which are not yet on the market, and/or;
- polypeptides which have a potential or assumed role in a metabolic or a biological pathway, which renders said polypeptides potentially useful as therapeutic agents. In order to determine if a polypeptide has a potential or assumed role in a metabolic or biological pathway, one may refer to biological, physiological, epidemiological, medical and clinical data made available to the experimenter with regard to the polypeptide or the nucleotide sequence thereof.

As an example, IFNα-2 is a polypeptide already used on the market, whereas IFNα-21 or IFNα-17, which belongs to the same gene family, are developed in the industry as therapeutic agents.

The nucleotide sequence of said preselected gene with therapeutic potential is usually available in nucleotide sequence databases like those run by these official institutions: EMBL (European Molecular Biology Laboratory; Meyerhofstrasse 1 D-69117 Heidelberg Germany), the NCBI (National Center for Biotechnology Information; National Library of Medicine Building 38A Bethesda, MD 20894 US) and DDBJ (DNA DataBase of Japan; 1111 Yata, Mishima, Shizuoka 411-8540, JAPAN).

A **"related gene"** of a preselected gene with therapeutic potential can be any gene belonging to the same gene family, as defined hereunder, as said preselected gene with therapeutic potential.

Two genes are considered as belonging to the same "**gene family**" if
- they display either enough nucleotide sequence identity,
- they code for polypeptides with enough amino acid sequence homology, and/or;
- they share a common amino acid pattern, which characterizes said family to permit their classification as members of a group of related genes.

The members of a gene family share the same or similar biological function(s) and have usually arisen from a common ancestor by gene duplication or amplification and have diverged subsequently by mutations.

The fact that a gene belongs to the same gene family as another gene is generally provided by the scientific literature, but also in the international nucleotide sequence databases such as those run by the official institutions previously mentioned. This information is usually further commented with respect to the gene's products in protein databases such as Swissprot® that is available at SwissProt, EMBL Outstation - European Bioinformatics Institute, Welcome Trust Genome Campus, Hinxton, Cambridge CB10 1SD, UK.

Alternatively, in particular if all the members of a gene family are not provided by the scientific literature, the one skilled in the art may determine if a gene belongs to a gene family, and to which one, by running a sequence homology search in an appropriate nucleotide or protein database like the one quoted above using a tool such as "blast" (basic local alignment search tool), which is well known by the one skilled in the art. The matches with the best score will correspond to members of said family. For example, the one skilled in the art may also use the software based on this principle which performs such a homology search, and which is available on the internet site http://www.ebi.ac.uk/clustr.

A gene belonging to the same gene family as the preselected gene with therapeutic potential is not necessarily reported in the literature as having a therapeutic potential.

The following gene families are given as examples: the family of gene encoding ribosomal RNA, the gene family encoding the Alpha-globins, the family encoding the Beta-globins, the gene family encoding the human Growth Hormone, the gene family encoding the Actin proteins, the gene family encoding the serine proteases, the gene family encoding the Vitellogenins, the gene family encoding the major histocompatibility antigens. As far as cytokines are concerned, it is usually considered that proteins displaying similar sequences characterized by more than 50 % amino acid identity, are encoded by genes belonging to the same family. These families include, among others the TGF-beta (T-cells Growth Factor) gene family, the EGF (Epithelial Growth Factors) gene family, the VEGF (Vascular Endothelial Growth Factor) gene family, the FGF (Fibroblast Growth Factors), the gene family encoding the Chemokines, the gene family encoding the neurotrophins. The IFNα (Interferons-alpha) constitute another cytokine gene family, that comprises at least 23 members like IFNα-2, IFNα-5, IFNα-17 and IFNα-21.

**"Polynucleotide"** is defined as a polyribonucleotide or a polydeoxribonucleotide that can be a modified or non-modified RNA or DNA.

The term polynucleotide includes, for example, single stranded or double stranded DNA, DNA composed of a mixture of one or several single stranded region(s) and of one or several double stranded region(s), single stranded or double stranded RNA, or RNA composed of a mixture of one or several single stranded region(s) and of one or several double stranded region(s). The term polynucleotide may also include RNA and/or DNA including one or several triple stranded regions. By polynucleotide are equally understood DNA and/or RNA containing one or several bases modified for reasons of stability or for other reasons. By modified base is understood, for example, the unusual bases such as inosine.

A **"Natural allelic variant"** of a gene is a polynucleotide present at the same locus as said gene, said polynucleotide and said gene differing in their nucleotide sequences by at least one non-synonymous coding genetic variation in the nucleotide sequence of said gene.

In the sense of the invention, a natural allelic variant of a gene may result from a change in the nature of one or more nucleotides, a deletion, an insertion or a repetition of one or more nucleotides in the nucleotide sequence of said gene.

A non-synonymous coding genetic variation is a polymorphism in the coding sequence of a nucleotide sequence that involves a modification of at least one amino acid(s) in the sequence of amino acids encoded by this nucleotide sequence. In this case, the genetic variation results in a variation in the amino acid sequence of the natural polypeptide.

**"Polypeptide"** is defined as a peptide, an oligopeptide, an oligomer or a protein comprising at least two amino acids joined to each other by a normal or modified peptide bond, such as in the cases of the isosteric peptides, for instance.

A polypeptide can be composed of amino acids other than the 20 amino acids defined by the genetic code. A polypeptide can equally be composed of amino acids modified by natural processes, such as post-translational maturation processes, which are well known to a person skilled in the art. Such modifications are fully detailed in the literature. These modifications can appear anywhere in the polypeptide: in the peptide skeleton, in the amino acid chain or even at the carboxy- or amino-terminal ends.

A polypeptide can be branched following an ubiquitination or be cyclic with or without branching. This type of modification can be the result of natural post-translational processes that are well known to a person skilled in the art. Such modifications are fully detailed in the literature: PROTEINS-STRUCTURE AND MOLECULAR PROPERTIES, 2^{nd} Ed., T. E. Creighton, New York, 1993; POST-TRANSLATIONAL COVALENT MODIFICATION OF PROTEINS, B. C. Johnson, Ed., Academic Press, New York, 1983; Seifter et al. "Analysis for protein modifications and nonprotein cofactors", Meth. Enzymol. (1990) 182: 626-646; and Rattan et al. "Protein Synthesis: Post-translational Modifications and Aging", Ann NY Acad Sci (1992) 663: 48-62.

A polypeptide encoded by a natural allelic variant of a gene may have an increased, reduced or suppressed biological activity in comparison with the polypeptide encoded by another natural allelic variant of the same gene.

A polypeptide encoded by a natural allelic variant of a gene can equally have a biological activity whose nature is changed in comparison with that of the polypeptide encoded by another allelic variant of the same gene.

An **"activity test"** designates any experiment performed to evaluate a given biological or pharmacological activity, or a biochemical, biophysical and/or physico-chemical property of therapeutic agent, which may consist of a polypeptide. An activity test may reflect the beneficial activity sought in a given therapeutic application or an adverse effect, as for example, toxicity.

The activity test for a given therapeutic agent may, for example, enable to determine its proliferative or anti-proliferative (anti-tumoral) activity, its affinity or absence of affinity to a ligand and/or a receptor, its immunomodulatory effect, its antiviral activity, its effects on cell differentiation. It may also enable to determine if the therapeutic agent may induce fever, immunogenicity or to evaluate the effect of the polypeptide on heart rate, blood pressure, appetite, hair loss, and depression. The activity test may also enable to determine differences in the level of gene expression or in the half-life of a polypeptide. Physico-chemical properties may refer, for example, to solubility of a therapeutic agent into a medium, its compatibility with a given excipient, or its resistance to proteolysis, for example. The literature, or his own knowledge, on the preselected gene will provide the one skilled in the art with the different activity tests that can be performed and with the standard protocols that are suitable to carry out the activity tests relevant in regard to a given therapeutic application.

A **"therapeutic index"** is a value that reflects the suitability of a therapeutic agent for a given therapeutic application. This value is generally a numerical value, but it can be also a quantitative value which can be arbitrary attributed by an experimenter. This value is generally based on the results obtained from at least two activity tests, as defined above. For instance, the "therapeutic index" of a therapeutic agent corresponds to the balance between the benefits of said therapeutic agent for a given therapeutic application and the adverse effects of said therapeutic agent. The benefits of a therapeutic agent are related to its therapeutic action or efficacy, which may be evaluated by *in vitro* and/or *in vivo* assays measuring the biological and pharmacological activity of said therapeutic agent required for the intended therapeutic application. The adverse effects of a therapeutic agent may correspond to the toxicity evaluated *in vitro* and/or *in vivo* in different biological models.

Usually the therapeutic index of a polypeptide is determined with respect to a product of reference, as defined below.

**"Therapeutic index of reference"** usually relates to the therapeutic index, as defined above, of a product of reference. The product of reference can be:
- a therapeutic agent already on the market;
- a therapeutic agent not yet on the market but developed in the industry in view to be marketed.
- any allelic variant of a preselected gene with therapeutic potential, as defined above.

Said product of reference can be of variable nature such as a chemical compound, a natural or synthetic polypeptide, a polynucleotide, for example.

Usually, the product of reference is chosen because it is known to be useful in a specific therapeutic application, in which it is believed that the polypeptides to be carried out in the method of the present invention are also useful.

The determination of the therapeutic index of reference usually requires that the experimenter carries out, simultaneously or not, the same protocol or substantially the same protocol for the product of reference and for the polypeptides that have to be compared.

As an example, interferon IFNα-2 can be a product of reference with regard to polypeptides encoded by natural allelic variants of genes belonging to the interferon alpha gene family.

Alternatively, the therapeutic index of reference can be established from data available with respect to at least one product of reference, which data can be found in the scientific literature or from clinical data. In this case the activity tests for all the polypeptides that are investigated, are usually performed according to the protocols provided by said scientific literature or said clinical data.

The natural allelic variants of a preselected gene with therapeutic potential and/or of any related gene considered in the invention may originate from the locus of the preselected gene with therapeutic potential or from the locus of said related gene that belongs to the same gene family as said preselected gene with therapeutic potential.

According to the present invention the polypeptides selected in step a) are polypeptides encoded by natural allelic variants of one preselected gene with therapeutic potential and polypeptides encoded by natural allelic variants of at least one related gene, it being understood that the related gene is a gene belonging to the same gene family as the preselected gene with therapeutic potential.

When none of the polypeptides selected in step a) shows a therapeutic index higher than the therapeutic index of reference, then none of said polypeptides are retained as suitable therapeutic agents.

According to a preferred embodiment of the invention, the determination of the therapeutic index of the polypeptides selected in step a) is achieved by means of the following steps:
i) submitting the polypeptides selected in step a) to at least two activity tests;
ii) attributing a value to each polypeptide in direct relation with the results of said activity tests; and
iii) determining the therapeutic index of each polypeptide from the values attributed in step ii).

The above step ii) can be performed by first, arbitrary or not, attributing a preliminary value to the results of each activity test, which means that a relative value is given to the results of each activity test, this value being established by comparison with the results obtained for a product of reference. Step iii) can be performed by integrating said preliminary values attributed to the activity tests for each polypeptide, in a single significant value that reflects the suitability of said polypeptide for a given therapeutic application.

The determination of the therapeutic index may be done by applying coefficients to said preliminary values and values. In this case, the choice of the coefficients depends on the specifications sought by the experimenter considering a given therapeutic application. For example, one may consider that the results of a specific *in vitro* test should receive a lesser coefficient than the results of a relevant *in vivo* test.

At least one activity test performed to determine the therapeutic index of the polypeptides selected in step a), may be carried out by means of a gene expression vector carrying a polypeptide which encodes said polypeptides.

The carrying out of such vectors may be of particular interest in the case where *in vivo* or *in situ* gene expression is sought, for example when the polypeptide is produced within a cell, and/or for gene therapy.

The polypeptides, which are retained as therapeutic agents according to the method of the present invention, may have an identical, similar or different pharmacological profile in comparison with that of the product of reference used to determine said therapeutic index of reference.

Thus, the method of the invention may also provide new therapeutic agents having a pharmacological profile different from the pharmacological profile of the product of reference.

By pharmacological profile, it is meant here the pharmacological effect(s) of a therapeutic agent.

Consequently, the method of the invention may provide new therapeutic agents suitable for therapeutic applications, which are different from those therapeutic applications of the product of reference.

The amino acid sequences of the polypeptides selected in step a) may be very similar one from each other. Thus, sequences of their mature form may differ, when compared in pair-wise combination, by less than 20 amino acids, preferably by less than 10 amino acids, more preferably by a single amino acid.

The polypeptides carried out in the method of the present invention are preferably used under their mature form. The mature form is understood as the active form of the polypeptide.

Another object of the present invention is a gene expression vector comprising a polynucleotide encoding a polypeptide obtainable according to the method of the present invention.

Such a gene expression vector can be used as a therapeutic agent, in particular for gene therapy. As this is well known, gene therapy consists in introducing a therapeutic gene into a patient to treat one disease or disorder. The methods to introduce a therapeutic gene into a patient are well known by the one skilled in the art. They can be carried out *ex vivo* (consisting in extracting patient's cells, inserting the therapeutic gene into said cells using a vector, introducing the resulting cells back in the patient), *in vivo* (consisting in introducing, into the blood vessels of the patient, the therapeutic gene incorporated in a vector, which should specifically reach the target cells), *in situ* (consisting in introducing the therapeutic gene incorporated in a vector in the target tissue).

A gene expression vector according to the present invention comprises a vector carrying said polynucleotide, which vector can be selected among those known by one skilled in the art as being suitable for gene therapy. Such vectors can comprise sequences of retrovirus, adenovirus, AAV virus, herpes virus, Poxvirus, synthetic vectors, nude DNA, liposomes, biolistic, etc.

The method for administrating a gene expression vector according to the present invention to a patient in need thereof can be determined by one skilled in the art in function of the disease or disorder to be treated.

Other objects of the present invention consist of therapeutic agents comprising a polypeptide obtainable by the method described above, a polynucleotide encoding said polypeptide, and/or a gene expression vector comprising said polynucleotide.

Another object of the invention is to provide a therapeutic agent comprising:
- a derivative of a polypeptide obtainable according to the method of the invention, said derivative resulting from drug optimisation technologies such as PEGylation, glycosylation, succinylation. These technologies aim at further increasing the therapeutic index of said polypeptide, and/or;
- a derivative of a polynucleotide encoding a polypeptide obtainable according to the method of the invention, said derivative resulting from site directed mutagenesis, directed evolution technologies devoted to further increasing therapeutic index of the polypeptide encoded by said polynucleotide.

Optimisation technologies such as PEGylation and glycosylation are known to one skilled in the art. They are described for example in US patents N°5382657 and N°6340242 respectively. These technologies enable to increase the half-life and stability of the polypeptides provided by the method of the invention.

Another object of the present invention is a therapeutic agent comprising a recombinant polypeptide whose amino acid sequence comprises several, preferably all the natural genetic variations characterizing the polypeptides obtainable according to the method of the invention.

Indeed, when a polypeptide is retained as a therapeutic agent in step c) of the present invention, this can be correlated to at least one mutation in the amino acid sequence of said polypeptide, which mutation results from at least one variation between the polynucleotide sequence of the corresponding natural allelic variant and the polynucleotide sequence of the preselected gene with therapeutic potential or said related gene thereof. Said mutation and natural genetic variation are regarded as characterizing a polypeptide. Such a mutation or natural genetic variation can be found in other polypeptides retained as therapeutic agents according to the method of the invention.

All these mutations or natural genetic variations could be combined in one single polypeptide by using techniques well known to the one skilled in the art, such as recombination based technologies. It is expected thereby that such recombinant polypeptides combine all the benefits of each individual polypeptide and be useful as therapeutic agent in one or several therapeutic applications.

According to another embodiment of the invention, one or more polypeptides selected as therapeutic agents by the method of the invention, may be used in combination for manufacturing a medicine, in order to combine the benefits of each individual polypeptide.

More generally, the therapeutic agents described above can be used, either individually or in combination, for the manufacture of a medicine for the treatment of a patient in need thereof. Said therapeutic agents are carried out at a therapeutically effective amount, which can be determined by one skilled in the art.

Said medicine usually comprises at least one pharmaceutically acceptable excipient such as talc, arabic gum, lactose, starch, dextrose, glycerol, ethanol, magnesium stearate, cocoa butter, aqueous or non-aqueous vehicles, fatty substances of animal or vegetable origin, paraffinic derivatives, glycols, various wetting agents, dispersants or emulsifiers, preservatives.

The therapeutic agents can be employed alone or in combination with other therapeutic compounds like cytokines, interleukins or interferons, for instance.

The different formulations of the pharmaceutical compositions are adapted according to the mode of administration.

The pharmaceutical compositions can be administered by different routes of administration known to a person skilled in the art, in particular orally, percutaneously, rectally.

A later object of the invention concerns a method for treating a patient having genetic deficiencies comprising the administration of a therapeutically effective amount of the therapeutic agent previously defined, and a pharmaceutically acceptable carrier.

### EXPERIMENTAL SECTION

The present invention is illustrated by tests performed on the polypeptides encoded by natural allelic variants of three genes belonging to the interferon alpha gene family, here after designated "IFNα" : C122S IFNα-5, G45R IFNα-17, Q114H and V127D IFNα-21, K179E IFNα-21. The natural allelic variants of IFNα encoding these polypeptides have been identified and isolated as described in the inventor's patent applications No. PCT/FR02/01516, PCT/EP02/05229, PCT/EP02/04082 (extension of FR0104404). The polypeptides encoded by the natural allelic variants of IFNα are submitted to several activity tests for several activity tests, which permits evaluation of their suitability for given therapeutic applications. For each activity test, the results obtained with said polypeptides are also compared with those obtained with a product of reference consisting of the polypeptide encoded by the wild-type allelic variant of IFNα-2 (IFNα-2b) chosen as representative of the interferon molecule. This product is marked under the trademark Intron A® by Schering Plough.

### Example1: Antiproliferative activity test on human lymphoblasts of Daudi Burkitt's cell line

In order to study the anti-proliferative effects of C122S IFNα-5, G45R IFNα-17, Q114H and V127D IFNα-21, K179E IFNα-21, and compare them with those of the reference (wild-type IFNα-2), cells from human Daudi Burkitt's lymphoma cell line, hereinafter called "Daudi cells", are cultivated in the presence of concentrations of one of said polypeptides ranging from 0.001 to 10 ng/ml.

The results of the anti-proliferative activity measurements obtained for each polypeptide tested permitted to calculate the concentration of interferon inhibiting the cell proliferation by 50% (IC50 value). The IC50 values determined for each interferon are reported in Table I.

**Table I**

| **Interferon** | **IC50 (ng/ml)** |
|---|---|
| C122S IFNα-5 | 0.390 |
| G45R IFNα-17 | 0.006 |
| Q114H/V127D IFNα-21 | 0.413 |
| K179E IFNα-21 | 0.024 |
| Wild-type IFNα-2 | 0.048 |

These results clearly demonstrate that the interferons tested have an antiproliferative activity on Daudi cells. Furthermore, the G45R IFNα-17 has the highest capacity to inhibit Daudi cells proliferation, which is also higher than that of the wild-type IFNα-2. The K179E IFNα-21 has also a higher capacity to inhibit Daudi cells proliferation than that of the wild-type IFNα-2.

### Example 2: Antiviral activity tests

The IFNs play an important role in the antiviral defence in mammals. The IFN antiviral activity is partly due to IFNs induced enzymatic systems, such as:
- The 2'5' oligoadenylate synthetase, an enzyme which catalyzes the adenosine oligomer synthesis. These oligomers activate the RNase L, an endoribonuclease which destroy the viral RNA once activated.
- The Mx proteins (GTPases) which inhibit the synthesis and/or the maturation of viral transcripts. This activity is mainly exerted on the influenza virus.
- The PKR protein (or p68 kinase) which is activated by the double-stranded RNA. The activated PKR inhibits viral protein synthesis.

The IFNs antiviral activity is also induced by other mechanisms such as, in the case of retroviruses, the inhibition of viral particle entry into the cells, the replication, the binding, the exit of the particles and the infective power of viral particles.

Finally, the IFNs exert an indirect antiviral activity by modulating certain functions of the immune system, in particular by favoring the response to cellular mediation (including an increase in the expression of the MHC class I and II molecules, increase in IFN-gamma production, increase in the CTL activities, among others).

The antiviral activity of C122S IFNα-5, G45R IFNα-17, Q114H and V127D IFNα-21, and K179E IFNα-21 has been evaluated both in cell culture and in mouse model. Both tests have been carried out in parallel with wild-type IFNα-2 used as the product of reference.

### a) Antiviral activity in cell culture

This assay permits evaluation of the antiviral activity of C122S IFNα-5, G45R IFNα-17, Q114H and V127D IFNα-21, and K179E IFNα-21 in cell culture using the vesicular stomatitis virus (VSV), and comparison with that of the reference (wild-type IFNα-2).

To do so, WISH human epithelial cells were cultivated for 24 hours in the presence of decreasing concentrations of each interferon. Then, the cells were infected by the virus of vesicular stomatitis (VSV) during 24 to 48 additional hours and cell lysis was measured.

The antiviral effect of the different IFNα tested is determined by comparing the IC50 value corresponding to the IFN concentration inhibiting 50% of cell lysis induced by the VSV.

A similar experiment has been carried out three times, and the IC50 values measured in one representative experiment are presented in Table II

**Table II**

| **IFNα polypeptides** | **IC50 (ng/ml)** |
|---|---|
| C122S IFNα-5 | 17 |
| G45R IFNα-17 | 2 |
| Q114H/V127D IFNα-21 | 22 |
| K179E IFNα-21 | 25 |
| Wild-type IFNα-2 | 4 |

The results of this experimentation indicate that the interferons tested exhibit antiviral activity in cell culture. In particular, among the interferons tested, the G45R IFNα-17 possesses the highest antiviral activity in cell culture infected with VSV, which is also higher than that of wild-type IFNα-2.

### b) Antiviral activity in mouse model

This test is performed in EMCV (Encephalomyocarditis virus) mouse model.

Human IFNs exhibit dose-dependent antiviral activity in the mouse which is in general 100 to 1,000 fold less than that exhibited by the same amount of mouse IFN (Meister *et al.* (1986), J. Gen. Virol. 67, 1633-1644).

Intraperitoneal injection of mice with Encephalomyocarditis virus (EMCV) gives rise to a rapidly progressive fatal disease characterized by deleterious effect on central nervous system and encephalitis (Finter NB (1973), Front Biol. 2: 295-360). Mouse and human interferon-alpha have both been shown to be effective in protecting mice against lethal EMCV infection (Tovey and Maury (1999), J. IFN Cytokine Res. 19: 145-155).

Groups of 20, six-week old Swiss mice were infected intraperitoneally with 100 x LD₅₀ EMCV and treated one hour later, and then once daily for 3 days thereafter with 2 µg of each interferon. A negative control group was performed with animals having been treated with excipient only. The animals were followed daily for survival for 21 days.

Results are presented in Figure 1 and indicate that the relative survival rate of the mice which have been treated with interferons is much higher than the survival rate of the non-treated mice, demonstrating the antiviral activity, in mouse model, of the interferons tested. The antiviral activity exhibited by the G45R IFNα-17 and Q114H and V127D IFNα-21 in mouse model is higher than that observed for the mice which have been treated with wild-type IFNα-2. Moreover, among all the interferons tested, the G45R IFNα-17 exhibits the highest antiviral activity in mouse model.

### Example 3: Immunomodulatory activity tests

IFNs type I (IFN alpha and IFN beta) are able to modulate certain functions of the immune system. The immunomodulatory activity of the interferons will be evaluated in parallel on dendritic cell maturation and in mice previously inoculated with malignant Friend erythroleukemia cells.

### a) Effect on dendritic cell maturation.

Immunomodulatory activity of C122S IFNα-5, G45R IFNα-17, Q114H and V127D IFNα-21, and K179E IFNα-21 was first investigated on dendritic cells maturation and compared with that of the reference (wild-type IFNα-2).

To do so, dendritic cells were first generated from adult human peripheral blood monocytes cultivated in the presence of GM-CSF and IL-4 cytokines. After purification using a CD14+ cells purification kit, these dendritic cells were placed in presence of 100 ng/ml of each interferon, and their phenotype was determined by FACS analysis aiming at looking for the expression of the MHC class I and II molecules and the CD40, CD80, CD86, CD83 and CD1a cell membrane markers. The maturation state of these dendritic cells has also been compared with that obtained without IFNα (no IFNα) treatment to provide a negative control with non-stimulated dendritic cells, and with treatment with either TNF-α (Tumor Necrosis Factor-α, 100 ng/ml) or LPS (Lipopolysaccharides, 1 µg/ml), to provide positive controls.

The median value of the measures of fluorescence intensity (FACS) for each marker, expressed as arbitrary unit, are presented in Table III.

**Table III**

| | **HLA ABC** | **HLA DR** | **CD40** | **CD80** | **CD86** | **CD83** | **CD1a** |
|---|---|---|---|---|---|---|---|
| No IFNα | 64 | 133 | 24 | 25 | 14 | 15 | 26 |
| LPS | 188 | 325 | 567 | 151 | 67 | 17 | 126 |
| TNFα | 72 | 209 | 355 | 49 | 9 | 13 | 181 |
| C122S IFNα-5 | 136 | 217 | 621 | 132 | 58 | 16 | 113 |
| Q114H V127D IFNα-21 | 68 | 122 | 163 | 46 | 8 | 8 | 191 |
| G45R IFNα-17 | 80 | 192 | 213 | 44 | 25 | 11 | 149 |
| K179E IFNα-21 | 181 | 322 | 491 | 87 | 44 | 14 | 127 |
| Wild-type IFNα-2 | 87 | 281 | 331 | 76 | 45 | 15 | 155 |

The results of this test demonstrate that the capacity to stimulate dendritic cell maturation varies according to the interferon tested. In particular, the C122S IFNα-5 and the K179E IFNα-21 exhibit the highest capacity to stimulate dendritic cell maturation, the activity of both polypeptides is higher than that of wild-type IFNα-2.

### b) Effect on survival of mice previously inoculated with malignant Friend erythroleukemia cells

IFNα have been shown to be equally effective in protecting mice against the growth of a clone of Friend leukemia cells resistant to the direct anti-proliferative activity of IFNα *in vitro* as against IFN sensitive parental Friend leukemia cells (Belardelli *et al.,* Int. J. Cancer, 30, 813-820, 1982; Belardelli *et al.*, Int. J. Cancer, 30, 821-825, 1982), reflecting the importance of indirect immune mediated mechanisms in the anti-tumoral activity of IFNα.

The following experimentation permits evaluation of the anti-tumoral activity of C122S IFNα-5, G45R IFNα-17, Q114H and V127D IFNα-21, and K179E IFNα-21 in mice previously inoculated with Friend erythroleukemia cells, and comparison with that of the reference (wild-type IFNα-2).

To do so, groups of 12 six-week old DBA/2 mice were inoculated intraperitoneally with 100,000 IFN resistant Friend leukaemia cells (3C18) (20,000 LD₅₀) and treated one hour later and then once daily for 21 days thereafter with 2.0 µg of each interferon or an equivalent volume of excipient alone. The animals were then followed daily for survival, for 70 days.

The results of this experiment, presented in Figure 2, indicate that, compared with mice which have not been treated with IFNα, treatment of mice with any of C122S IFNα-5, G45R IFNα-17, Q114H and V127D IFNα-21, and K179E IFNα-21 results in an increase in the number of mice surviving 70 days after inoculation with highly malignant Friend erythroleukemia cells (FLC). In particular, the survival of FLC inoculated mice is the highest after treatment with K179E IFNα-21 and is also very high with Q114H and V127D IFNα-21, the survival rate measured after treatment with one of these two polypeptides being higher than after treatment with wild-type IFNα-2.

### Example 4: Toxicity activity tests

Like most other cytokines the interferons alpha are produced to act locally in the body. Therefore, their systemic pharmacological use induces toxic effects. The most consistent acute effect reported during interferon alpha therapy, after each administration of interferon, in a majority of patients is the "flu syndrome" that results in fever, fatigue, weight and appetite loss. This syndrome has also been reported, after each administration, in Rhesus monkeys, which are considered to be very close to humans. Moreover, cardiologic effects have also been reported as adverse effects caused by interferons alpha administered in systemic.

Therefore, safety pharmacological study was performed in conscious Rhesus monkeys monitored by telemetry to test the effect on temperature, arterial blood pressure, and heart rate of an acute subcutaneous administration of each interferon.

In this model, for each animal, body temperature and haemodynamic parameters were recorded two hours before the administration of interferon and for 24 hours following the administration. For each of the interferons tested, the doses of interferon administered subcutaneously were 1 and 10 µg/kg in two animals and 20 µg/kg in one animal. A negative control corresponding to administration of excipient only was also performed demonstrating that the administration of vehicle had no effect on body temperature (the first clinical sign usually observed), arterial blood pressure, and heart rate.

As indicated in figure 3, the subcutaneous administration of 1 µg/kg, 10 µg/kg or 20 µg/kg of C122S IFNα-5 had no significant effect on body temperature in monkey. As shown in figure 3, among the five interferons tested, the C122S IFNα-5 exhibits the lowest toxicity, which is estimated by the increase in body temperature of the monkey. Compared with the other interferons tested, the K179E IFNα-21 exhibits a low toxicity too. Both the C122S IFNα-5 and K179E IFNα-21 exhibit a lower toxicity in monkeys than the wild-type IFNα-2.

Similarly, at all tested doses, the administration of any of the interferons tested has no significant effect on arterial blood pressure and heart rate in monkey.

### Example 5: Therapeutic index determination

The therapeutic index of each polypeptide tested in examples 1 to 4 was determined on the basis of the results obtained from the activity tests described in said examples 1 to 4.

In fact, for each tested polypeptide, three different therapeutic index with regard to the three investigated therapeutic applications were determined, as follows:
- an antiviral therapeutic index related to the antiviral application, which was determined using the results of the antiviral activity tests and the toxicity activity tests;
- an antiproliferative therapeutic index related to antiproliferative application, which was determined using the results of the antiproliferative activity tests and the toxicity activity tests ;
- an imunomodulatory therapeutic index related to the immunomodulatory application, which was determined using the results of the immunomodulatory activity tests and those of the toxicity activity tests.

Each therapeutic index reflects therefore the possible use of a tested polypeptide in a given therapeutic application.

More particularly, in order to determine said therapeutic index, one has attributed a preliminary value comprised between -3 and +3, to each result obtained for each polypeptide in said activity tests.

These preliminary values were attributed arbitrarily by the experimenter in the following manner:

When the result obtained for a polypeptide in one activity tests is equal or close to the one obtained for the product of reference (the wild-type IFNα-2), the attributed preliminary value was 0; as the preliminary values for the polypeptides tested were determined in comparison with the results obtained with the product of reference, the preliminary values for said product of reference are all necessary equal to 0. When said result was regarded as slightly, strongly or very strongly above the results of the product of reference, the attributed preliminary value was respectively +1, +2 and +3. Similarly, when said result was slightly, strongly or very strongly below the result of the product of reference, the attributed preliminary value was of respectively, -1, -2 and -3. Furthermore, the experimenter has arbitrarily applied coefficients to each activity tests, which coefficients were affected to preliminary values determined as described above.

Then, for each therapeutic application, one has determined an average value by adding the preliminary values of each activity test, affected with their respective coefficients. The results so obtained are described in Table IV here below.

Afterwards, for each polypeptide tested, the therapeutic index was determined by subtracting the toxicity average value, which was furthermore affected by a coefficient of 2/3, to the sum of the average values obtained from the other activity tests.

The coefficients so applied depend on the impact attributed by the experimenter, of a given activity test on the therapeutic application, which is reflected through the therapeutic index to be determined.

For example, a coefficient of 0,4 was affected to the antiviral activity test performed in cell culture, while a coefficient of 0,6 was affected to the antiviral activity test performed in mice. This was done so because it has been considered by the experimenter that the test performed in cell culture was less relevant than the one performed in mice, with regard to the antiviral application.

The therapeutic index of reference being determined from the preliminary values for the product of reference, and which are all equal to 0, the therapeutic index, in this present case, is equal to 0.

The therapeutic indexes of reference thus obtained are shown in table V hereafter.

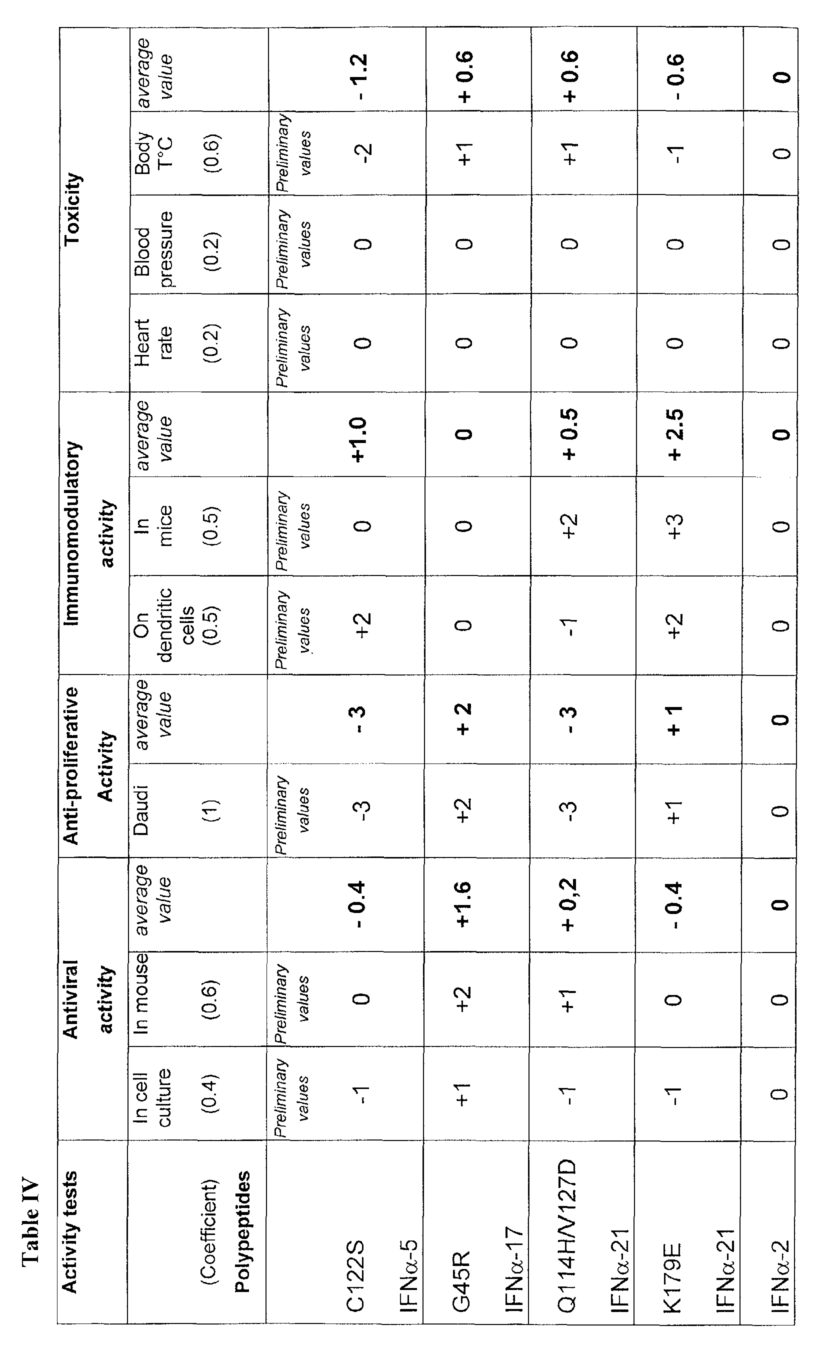

**Table V**

| | **Therapeutic index** | | |
|---|---|---|---|
| | **Antiviral application** *values* | **Antiproliferative application** *values* | **Immunomodulatory application** *values* |
| C122S IFNα-5 | +0.4 | -2.2 | +**1.8** |
| G45R IFNα-17 | +**1.2** | +**1.6** | -0.4 |
| Q114H/V127D IFNα-21 | - 0.2 | - 3.4 | + 0.1 |
| K179E IFNα-21 | 0 | +**1.4** | +**2.9** |
| IFNα-2 (reference) | 0 (index of reference) | 0 (index of reference) | 0 (index of reference) |

The results so obtained demonstrate, for the first time, a dissociation between the different kinds of activities of a pleiotropic polypeptide, which means that each one of these activities may be differently affected by a natural genetic variation i.e. the polypeptide encoded by a natural allelic variant of the interferon may have a higher immunomodulatory activity, for example, but a lesser or similar toxicity in comparison with the wild-type interferon on the market. As a consequence, the method of the invention can provide new therapeutic agents with new pharmacological profiles.

These results also demonstrate that the natural allelic variants of a preselected gene with therapeutic potential and genes belonging to the same gene family constitute an actual source for molecules, which can be efficiently used as therapeutic agents. Indeed, these results indicate that:
- therapeutic index of G45R IFNα-17 and therapeutic index of C122S IFNα-5 are higher than that of wild-type IFNα-2 in therapeutic applications where antiviral activity is required. In particular G45R IFNα-17 has the highest therapeutic index for said therapeutic applications. Thus, C122S IFNα-5 and, still more, G45R IFNα-17, or any other polynucleotide encoding said polypeptides, may be used as therapeutic agents to treat diseases or disorders requiring an antiviral activity;
- therapeutic index of G45R IFNα-17 and therapeutic index of K179E IFNα-21 are higher than the reference and are the highest and second highest, respectively, in therapeutic applications where antiproliferative activity is required. Thus, one of these two polypeptides or both, or any polynucleotide encoding said polypeptides, may be used as therapeutic agents to treat diseases or disorders requiring an antiproliferative activity; and
- therapeutic index of K179E IFNα-21 and therapeutic index of C122S IFNα-5 are higher than the reference and are the highest and second highest, respectively, in therapeutic applications where immunostimulatory activity is required. Thus, K179E IFNα-21 and/or C122S IFNα-5, or any polynucleotide encoding said polypeptides, may be used as therapeutic agents to treat diseases or disorders requiring an immunostimulatory activity.

In addition, the method of the invention provides new therapeutic agents suitable for new therapeutic applications. Indeed, the results disclosed in these examples demonstrate that, in contrast to the product of reference which is known to be used to treat melanoma (requiring antiproliferative activity) and hepatitis C (requiring antiviral activity) but not to treat diseases or disorders requiring immunomodulatory activity, some therapeutic agents selected with the method of the invention i.e. the K179E IFNα-21 and C122S IFNα-5 polypeptides have a higher therapeutic index than the reference in therapeutic applications where immunostimulatory activity is required.

## Claims

1. A method for providing new therapeutic agent(s), **characterized in that** it comprises the following steps:
a) selecting at least one polypeptide encoded by a natural allelic variant of one preselected gene with therapeutic potential and/or of at least one related gene(s) thereof;
b) determining the therapeutic index of the polypeptide(s) selected in step a); and
c) retaining as a therapeutic agent(s), the polypeptide(s) selected in step a), whose therapeutic index, as determined in step b), is higher than a therapeutic index of reference.

2. The method according to claim 1, **characterized in that** at least 2, preferably at least 4 polypeptides are selected in step a).

3. The method according to any one of claims 1 and 2, **characterized in that** it further comprises a step d), wherein only the polypeptide(s) retained in step c) which has (have) the highest or second highest therapeutic index is (are) selected as therapeutic agent(s).

4. The method according to any one of claims 1 to 3, **characterized in that** step b) comprises the following steps:
i) submitting the polypeptides selected in step a) to at least two activity tests;
ii) attributing a value to each polypeptide in direct relation with the results of said activity tests; and
iii) determining the therapeutic index of each polypeptide from the values attributed in step ii).

5. The method according to any one of claims 2 to 4, wherein the polypeptides selected in step a) are
- polypeptides encoded by natural allelic variants of one preselected gene with therapeutic potential; and
- polypeptides encoded by natural allelic variants of at least one related gene.

6. The method according to any one of claims 1 to 5, wherein said natural allelic variants originate from the same species.

7. The method according to claim 6, wherein said the natural allelic variants originate from the human species.

8. The method according to any one of claims 1 to 4, wherein the polypeptides selected in step a) are polypeptides encoded by natural allelic variants of one single gene that can be either the preselected gene with therapeutic potential or one related gene thereof.

9. The method according to any one of claims 1 to 8, wherein the polypeptides selected in step a) are under their mature form.

10. The method according to any one of claims 1 to 9, wherein the amino acid sequences of the polypeptides selected in step a) differ, when compared in pair-wise combination, preferably by less than 20 amino acids, more preferably by less than 10 amino acids, still more preferably by a single amino acid, with regard to the polypeptide mature form.

11. The method according to any one of claims 1 to 10, wherein said preselected gene with therapeutic potential is a gene encoding for a cytokine.

12. The method according to claim 4, wherein at least one activity test is carried out by means of a gene expression vector carrying a polynucleotide, which encodes the polypeptide, selected in step a).

13. A therapeutic agent **characterized in that** it is obtainable by the method according to any one of claims 1 to 12.

14. A therapeutic agent comprising a polypeptide obtainable according to the methods of any one of claims 1 to 12.

15. A therapeutic agent comprising a polynucleotide encoding a polypeptide obtainable according to any one of claims 1 to 12.

16. A gene expression vector comprising a polynucleotide encoding a polypeptide obtainable according to the method of any one of claims 1 to 12.

17. A therapeutic agent comprising:
- a derivative of a polypeptide obtainable according to any one of claims 1 to 12, said derivative resulting from drug optimisation technologies such as PEGylation, glycosylation, succinylation, to further increase the therapeutic index of said polypeptide, and/or;
- a derivative of a polynucleotide encoding for a polypeptide obtainable according to any one of claims 1 to 12, said derivative resulting from site directed mutagenesis, directed evolution technologies, to further increase the therapeutic index of the polypeptide encoded by said polynucleotide.

18. A therapeutic agent comprising a recombinant polypeptide whose amino acid sequence comprises several, preferably all the natural genetic variations characterizing the polypeptides obtainable according to any one of claims 1 to 12.

19. The use of a therapeutically effective amount of a therapeutic agent according to any one of claims 12 to 15 and 17 to 18, or a gene expression vector according to claim 16, for the manufacture of a medicine for the treatment of a patient in need thereof.

20. The use of the method according to any one of claims 1 to 12 to provide new therapeutic agents with new pharmacological profiles, and/or to provide new therapeutic agents for new therapeutic applications, with respect to a product of reference.
